# EUROPÄISCHE PATENTANMELDUNG

(11) **EP 1 810 716 A1**
(43) Veröffentlichungstag der Anmeldung: **25.07.2007**
(21) Anmeldenummer: 05112919.5
(22) Anmeldetag: 23.12.2005
(51) Int. Cl.: A61Q 19/04, A61K 8/60, A61Q 17/04, A61P 17/00, A61K 8/49, A61K 8/64

(54) **Verfahren zur Bräunung der Haut**

(71) Anmelder: Deutsches Zentrum für Luft- und Raumfahrt e.V., 51147 Köln (DE)
(72) Erfinder: Gerzer, Rupert, 53113 Bonn (DE); Ivanova, Krassimira, 51143 Köln (DE)
(74) Vertreter: Jönsson, Hans-Peter

(57) **Zusammenfassung**

Gegenstand der Erfindung ist ein Verfahren zur Bräunung der menschlichen oder tierischen Haut durch eine Steigerung des intrazellulären Spiegels von cGMP oder cGMP-Analoga sowie die Verwendung eines hierfür geeigneten Stoffes oder Stoffgemisches zur Therapie von Pigmentstörungen, zur Therapie von Melanin-Stoffwechselstörungen und/oder zur Bräunung der Haut.

## Beschreibung

Gegenstand der Erfindung ist ein Verfahren zur Bräunung der menschlichen oder tierischen Haut durch eine Steigerung des intrazellulären Spiegels von cGMP oder cGMP-Analoga sowie die Verwendung eines hierfür geeigneten Stoffes oder Stoffgemisches zur Therapie von Pigmentstörungen, zur Therapie von Melanin-Stoffwechselstörungen und/oder zur Bräunung der Haut.

### Hintergrund

Gebräunte Haut gilt insbesondere in Ländern wie Deutschland mit überwiegend hellhäutiger Bevölkerung in verschiedenen Bevölkerungsschichten als Zeichen von Weltläufigkeit, Offenheit und Dynamik und als Inbegriff modernen Lebensstils und wird deshalb von vielen angestrebt. Insbesondere in den Wintermonaten ist die Standardmethode, gebräunte Haut zu erreichen - falls man keine Fernreise antreten kann - in ein Sonnenstudio zu gehen und sich dort UV-Licht (insbes. UV-A-Licht) auszusetzen und eine schnelle Hautbräunung zu induzieren.

Es existieren auch Alternativverfahren wie beispielsweise eine Hautbräunung mittels Auftrag von Substanzen wie Dihydroxyaceton auf die Haut, das dann resorbiert wird und durch Sonnenlicht einen gelblich-braunen Farbton annimmt. Dies sieht dann wie sonnengebräunte Haut aus. Da die entsprechenden Hautzellen nach ca. 4 Tagen abgeschliffen werden, hält diese "Bräunung" nur für wenige Tage.

Das direkte, physiologische Verfahren ist, sich Sonnenlicht auszusetzen, dessen UV-Anteile (insbes. UV-B) eine physiologische Reaktion in Gang setzen, so dass vermehrt Melanin gebildet wird, das dann in Melanozyten vermehrt vorhanden ist. Dadurch wird einerseits die Haut vor Sonnenlicht geschützt und andererseits wird die Haut wegen der Bräune als schön empfunden.

Ein Verfahren, welches die durch Sonnenlicht induzierte vermehrte physiologische Melaninproduktion in der Haut stimuliert, hätte den Vorteil, dass es zum Beispiel bei UV-empfindlichen Personen vor einer zu erwartenden erhöhten UV-Exposition angewandt werden könnte, ohne dabei wie zum Beispiel durch das Aufsuchen eines Sonnenstudios die Nachteile einer UV-Exposition (Hautalterung, Abwehrschwächung, Krebsrisiko, Allergiesicherung in Richtung Photodermatosen) in Kauf nehmen zu müssen.

Durch Produktion von Melanin in Melanocyten nimmt die Haut eine braune Färbung an. Melanin entsteht in den Melanocyten durch die Umsetzung von Tyrosin mit Hilfe von Tyrosinase zu Dopaquinon, welches wiederum mit Hilfe eines TRYP-1 Proteins (tyrosinase-related protein) über Zwischenstufen zu Melanin umgesetzt wird.

Weiterhin ist bekannt, dass ein hoher Spiegel von cGMP (zyklisches 3',5'-Guanosinmonophosphat) zur Stimulierung der Tyrosinase und des "tyrosinase related protein" 1 (TRYP-1) führt ( Romero-Graillet, C., J. Biol. Chem. 1996, 271, 28052-28056; Sasaki, M. et al., Pigment Cell Res. 2000, 13:248-52).

cGMP wird aus GTP (Guanosintriphosphat) mit Hilfe von Guanylatzyklase (GC) gebildet. Es ist bereits bekannt, dass Guanylatzyklase auf verschiedene Arten aktiviert werden kann. Dies sind zum einen NO-abhängige Wege, wie beispielsweise durch organische Nitrate (z.B. Nitroglycerin), NONOates (z.B. DETA-NO), S-Nitroso-glutathion, Natriumnitroprussid, und zum anderen NO-unabhängigen Wege, wie beispielsweise Aktivatoren der löslichen Guanylatzyklase (z.B. YC-1, BAY 41-2272 und BAY 41-8543) oder Aktivatoren der membrangebundenen Guanylatzyklase (z.B. Natriuretische Peptide).

Stickstoffmonoxid (NO) spielt eine Schlüsselrolle in physiologischen und pathophysiologischen Prozessen in verschiedenen Geweben wie z. B. Entzündungsprozessen und Hautkrebs. Die Synthese von NO erfolgt mit Hilfe spezifischer Enzymsysteme, die durch extra- und intrazelluläre Signale aktiviert werden. Ausgangspunkt der Synthese von NO ist die Aminosäure Arginin, die von der NO-Synthase durch eine Redoxreaktion unter Mitwirkung von O₂ und NADPH₂ zu NO und Citrullin umgesetzt wird.

Die biologische Bedeutung von NO als "messenger" Molekül wurde ursprünglich im Zusammenhang mit der Kontraktion und Relaxation der Blutgefäße erkannt. Die Zunahme der cGMP-Konzentration in vaskulären glatten Muskulaturzellen ist mit einer Aktivierung der cGMPabhängigen zytosolischen Proteinkinase (PKG) verbunden. Dies führt anschließend zur Phosphorylierung zahlreicher intrazellulärer Proteine.

Die lösliche GC ist auch das Ziel von NO-Donoren, die seit längerem erfolgreich in der Therapie der Angina pectoris eingesetzt werden. In den letzten Jahren wurde noch zusätzlich über direkte Aktivatoren der löslichen GC berichtet. Als erste dieser Substanzen wurde 3-(5'-hydroxymethyl-2'-furyl)-1-benzylindazol (YC-1) beschrieben (Wu, C.C. et al., British J. Pharmacol. 1995, 116, 1973-1978). YC-1 erhöht den cGMP-Gehalt in Thrombozyten und hat eine antiaggregatorische (A. Friebe et al., Mol. Pharmacol. 1998, 53, 23-127) und vasodilatatorische Wirkung (J. Galle, British J. Pharmacol. 1999, 127, 195-203). Der Wirkmechanismus dieser Substanz ist auf direkte Aktivierung der löslichen GC und nicht auf die Freisetzung von NO zurückzuführen (J.P.Stasch et al., Nature 2001, 410(6825), 212-215).

cGMP wird durch Phosphodiesterasen zu GMP hydrolisiert. Dieser Abbau von cGMP kann durch Hemmung von Phosphodiesterasen verlangsamt werden. In der Literatur sind in einem anderen Zusammenhang (Therapie von Erektionsstörungen) einige dieser Inhibitoren bekannt (Zaprinast, Sildenafil oder auch Vardenafil).

Weiterhin kann der Export von cGMP aus der Zelle gehemmt werden. Die Untersuchung des aktiven cGMP-Transportsystems in humanen Erythrozyten zeigte, dass die zelluläre Extrusion des cGMP ein einseitig wirkender ATP-abhängiger Prozess ist (beispielsweise in E. Sundkvist et al., Biochem. Biophys. Acta. 2000, 1463, 121-130), der sich durch einen nichtselektiven Transporthemmstoff von organischen Anionen, "Probenecid", inhibieren lässt. Dies deutet darauf hin, dass der ATPaseabhängige Export durch Transporter vermittelt wird, die als "multidrug resistance protein" (MRP) identifiziert wurden (siehe beispielsweise V.

Millul et al., Am. J. Physiol. 1996, 39, C1051-C1060). Als cGMSspezifische Transporter wurden MRP4 und MRP5 charakterisiert (Jedlitschky, G. et al., J. Biol. Chem. 2000, 275:30069-30074; Lai L. und Tan, T.M., Biochem. J. 2002, 361:497-503; van Aubel, R.A. et al., J. Am. Soc. Nephrol. 2002, 13:595-603).

Weiterhin ist bekannt, dass MRP4 und MRP5 durch Inhibitoren der Phosphodiesterasen (beispielsweise PDE5) wie beispielsweise Sildenafil gehemmt werden können.

Es wurde bereits gezeigt, dass eine Erhöhung der cGMP-Bildung mittels Stickstoffmonoxid-freisetzender Substanzen mit einer vermehrten Melaninanreicherung in normalen humanen Melanozyten korreliert (K. Ivanova et al., J. Invest. Dermatol. 2001, 116, 409-416).

Weiterhin wurde gefunden, dass UV-B Bestrahlung über die NO-Kaskade und dadurch erhöhte Spiegel von cGMP in Melanozyten zu erhöhter Melaninsynthese führt (C. Romero-Graillet, J. Biol. Chem. 1996, 271, 28052-28056).

DE 199 45 484 A1 beschreibt eine NO-freisetzende, topisch applizierbare Zusammensetzung zur Bräunung der Haut. Stickstoffmonoxid selbst kann jedoch mit Sauerstoffradikalen zu toxischen und hochkanzerogenen Radikalen reagieren, was insbesondere in Melanozyten wegen des Risikos von malignen Melanomen vermieden werden sollte.

Nachteile des Standes der Technik sind also, dass man sich entweder natürlichem oder künstlichem UV-Licht aussetzen muss, um "braun" zu werden, oder dass man nur eine chemisch induzierte Imitation erreicht, die nur für wenige Tage anhält und nichts mit dem körpereigenen Schutz gegen UV-Licht zu tun hat, der mit einer Bräunung einhergeht. Weiterhin sind die bekannten Verfahren zur Hautbräunung, welche sich die NO-Kaskade zunutze machen, nachteilig, da durch die Präsenz von NO in Melanozyten das Krebsrisiko erhöht wird.

Bislang ist eine Melanogenese in Melanocyten nicht ohne den Einsatz von UV-Licht, etwa durch Einsatz von Stickstoffmonoxid-unabhängigen Substanzen oder MRPs (multidrug-resistance proteins) bekannt.

Es ist also die Aufgabe der vorliegenden Erfindung, ein Verfahren zur Hautbräunung zur Verfügung zu stellen, welches die vorgenannten Nachteile des Standes der Technik überwindet.

### Kurzbeschreibung der Erfindung

Diese Aufgabe wird gelöst durch das erfindungsgemäße Verfahren zum Bräunen der Haut. Dieses erfindungsgemäße Verfahren umfasst das Applizieren eines Mittels auf oder in die Haut, welches eine oder mehrere Substanzen enthält, die eine Steigerung des intrazellulären Spiegels von cGMP oder cGMP-Analoga bewirken. Diese Substanzen sind vorzugsweise ausgewählt aus cGMP, zellpermeablen cGMP-Analoga, Inhibitoren von Phosphodiesterasen, Inhibitoren von Multidrug Resistance Proteins (MRPs), NO-unabhängigen Aktivatoren von Guanylatzyklase, und den pharmazeutisch akzeptablen Salzen derselben.

Gegenstand der vorliegenden Erfindung ist somit
(1) ein Verfahren zum Bräunen der menschlichen oder tierischen Haut umfassend das Applizieren eines Mittels auf oder in die Haut, welches eine oder mehrere Substanzen enthält, die eine Steigerung des intrazellulären Spiegels von cGMP oder cGMP-Analoga bewirken;
(2) die Verwendung des Mittels wie unter (1) definiert zur Bräunung der menschlichen oder tierischen Haut;
(3) die Verwendung des Mittels wie unter (1) definiert zur Herstellung eines Arzneimittels zur Therapie von Melanin-Stoffwechselstörungen oder Pigmentstörungen; und
(4) ein Mittel zur Bräunung der Haut wie unter (1) definiert.

### Detaillierte Beschreibung der Erfindung

Insgesamt geht es bei der vorliegenden Erfindung darum, mittels einer Steigerung der funktionellen cGMP-Spiegel unter Umgehung des Prinzips "Stickstoffmonoxid" den physiologischen Mechanismus der Melanin-Anreicherung zu stimulieren und dadurch ohne vermehrtes Sonnenlicht eine "physiologische" Hautbräunung zu erreichen, ohne gleichzeitig das Risiko einer durch Stickstoffmonoxid erhöhten Hautkrebsgefahr einzugehen.

Dies hat zum einen erhebliche Vorteile bezüglich des Lebensgefühls von Nutzern des erfindungsgemäßen Verfahrens und kann auch bei sonnenempfindlichen Menschen vor einer UV-Exposition einen natürlichen Schutz gegen UV-Licht aufbauen.

Die der Erfindung zugrundeliegende Aufgabe wird also durch Vermeidung von Stickstoffmonoxid gelöst, um eine vermehrte Melanineinlagerung in Hautzellen zu erreichen. Unter Umgehung von Stickstoffmonoxid selbst wird das Prinzip verwendet, über das Stickstoffmonoxid im Wesentlichen wirkt, nämlich eine Erhöhung der Spiegel von cGMP. Eine Erhöhung der cGMP-Spiegel ist also durch Vermeidung der Präsenz von Stickstoffmonoxid ohne erhöhtes Krebsrisiko erreichbar.

Einerseits können Aktivatoren löslicher Guanylatzyklase eingesetzt werden, die das Enzym stickstoffmonoxid-unabhängig aktivieren (J.P. Stasch et al., Nature 2001, 410(6825), 212-215). Alternativ kann auch eine Hemmung des cGMP-Abbaus in diesen Zellen zum Zuge kommen, wie sie mittels cGMP-Phosphodiesterasehemmung beim Wirkprinzip von Sildenafil ("Viagra^{®}") zum Einsatz kommt (M.A. Blount et al., Mol. Pharmacol. 2004, 66(1), 144-152). Eine weitere Methode ist die Hemmung der cGMP-Ausschleusung aus Zellen, die zu Anstiegen intrazellulärer cGMP-Spiegel führt (K. Ivanova, Pigmet Cell Res. 2004, 17, 471-479; K. Ivanova, J. Gravit. Physiol. 2002, 9, 271-272). Ferner können Stimulatoren anderer Guanylatzyklasen wie beispielsweise natriuretische Peptide oder Guanyline angewandt werden (M. Chinkers et al., Nature, 1989, 338(6210), 78-83; I.R. Forte et al., FASEB J. 1995, 9, 643-650) Schließlich können funktionelle, membrangängige cGMP-Analoga verwendet werden, die ebenso den Effekt induzieren.

Bevorzugte Ausführungsformen des Verfahrens (1) sind Verfahren, in denen das verwendete Mittel eine oder mehrere Substanzen ausgewählt aus der Gruppe umfassend cGMP, cGMP-Analoga, Inhibitoren von Phosphodiesterasen, Inhibitoren von Multidrug Resistance Proteins (MRPs), NO-unabhängigen Aktivatoren von Guanylatzyklase und die pharmazeutisch akzeptablen Salze derselben. In besonders bevorzugter Ausführungsform sind besagte Substanzen ausgewählt aus cGMP-Analoga, Phosphodiesterase-Inhibitoren und Guanylatzyklase-Aktivatoren. Von den cGMP-Analoga sind zellpermeable cGMP-Analoga bevorzugt, da diese im Gegensatz zu cGMP selbst die Zellmembranen passieren können. Sie können in der Zelle hydrolysiert werden, was zur Freisetzung von cGMP führt.

Bevorzugte cGMP-Analoga sind ausgewählt aus der Gruppe umfassend 8-PCPT-cGMP (8-(4-chlorophenylthio)guanosine 3',5'-cyclic monophosphate), 8-Bromo-cGMP und Monobutyryl-3,5-cGMP.

Die für das erfindungsgemäße Verfahren einsetzbaren Phosphodiesterase-Inhibitoren sind entweder spezifisch, d.h. sie inhibieren nur die cGMP-spezifischen Phosphodiesterasen von Typ V, oder unspezifisch. Bevorzugte unspezifische Inhibitoren von Phosphodiesterasen sind ausgewählt aus der Gruppe umfassend Koffein, 3-Isobutyl-1-Methylxanthin und Theophyllin. Bevorzugte spezifische Inhibitoren von Phosphodiesterasen sind ausgewählt aus der Gruppe umfassend Zaprinast, Trequinsin, Sildenafil, Tadalafil und Vardenafil.

Bevorzugte Aktivatoren von Guanylatzyklase sind ausgewählt aus der Gruppe umfassend 3-(5'-hydroxymethyl-2'-furyl)-1-benzylindazol (YC-1), natriuretische Peptide wie h-BNP 32 (brain natriuretic peptide), h-CNP 22 (C-type natriuretic peptide), α-ANP (atrial natriuretic peptide), Guanylin und Uroguanylin.

Als cGMP-Analogon wird besonders bevorzugt 8-PCPT-cGMP eingesetzt, da seine Lipophilie wesentlich höher ist als diejenige von anderen cGMP-Analoga wie 8-Bromo-cGMP oder von cGMP selbst. Erhöhte Lipophile bedeutet eine verbesserte Zellmembranpermeabilität (M.Y. Menshikov, Eur. J. Pharmacol. 1993, 245, 281).

Als Inhibitor von Phosphodiesterasen wird besonders bevorzugt Trequinsin eingesetzt.

Als Aktivator von Guanylatzyklase wird besonders bevorzugt 3-(5'-hydroxymethyl-2'-furyl)-1-benzylindazol (YC-1) eingesetzt.

Vorteilhafterweise wird im erfindungsgemäßen Verfahren (1) das Mittel auf die Haut, insbesondere als Creme, Salbe, Gel, Paste, Lotion oder Lösung, unverdünnt und/oder als Teil eines Pflasters appliziert. Dies gilt insbesondere dann, wenn das Mittel eines oder mehrere der erfindungsgemäßen cGMP-Analoga, Inhibitoren von Phosphodiesterasen und/oder Aktivatoren von Guanylatzyklase enthält.

Der Gehalt dieser Verbindungen oder anderer Substanzen, welche eine Steigerung des intrazellulären Spiegels von cGMP oder cGMP-Analoga bewirken, in der für die Applikation gewählten Formulierung richtet sich nach der Art der Formulierung, der Beschaffenheit und Vorbräunung der Haut, der zu erreichenden Dosis bzw. dem gewünschten Ergebnis, dem pharmazeutischen oder kosmetischen Anwendungsgebiet etc. Er kann vom Fachmann unter Berücksichtigung dieser und weiterer Faktoren und ggf. unter Zuhilfenahme von Routineversuchen zu Formulierungsparametern wie Löslichkeit und Stabilität der Verbindungen festgelegt werden.

cGMP-Analoga und Guanylatzyklase-Aktivatoren werden in flüssigen und cremigen Formulierungen bevorzugt in Konzentrationen von 400 µg/ml bis 1200 µg/ml, besonders bevorzugt von 700 µg/ml bis 900 µg/ml verwendet. In Pflastern können von 10 µg/cm² bis 50 µg/cm², bevorzugt von 30 µg/cm² bis 40 µg/cm² enthalten sein.

Wegen ihrer höheren biologischen Aktivität und der daraus resultierenden geringen notwendigen Dosis ist die bevorzugte Konzentration von spezifischen Inhibitoren von Phosphodiesterasen entsprechend niedriger anzusetzen. Sie beträgt in flüssigen und cremigen Formulierungen von 40 µg/ml bis 100 µg/ml, bevorzugt von 60 µg/ml bis 80 µg/ml. In Pflastern können von 20 µg/cm² bis 50 µg/cm², bevorzugt von 30 µg/cm² bis 40 µg/cm² enthalten sein. Die bevorzugte Konzentration von unspezifischen Phosphodiesterase-Inhibitoren ist zwei- bis zehnfach höher als die bevorzugte Konzentration der spezifischen Phosphodiesterase-Inhibitoren.

Werden mehrere der genannten Substanzen in einem Mittel verwendet, so sollte ihre Gesamtkonzentration 1200 µg/ml nicht überschreiten. Bei Anwesenheit eines oder mehrerer Inhibitoren von Phosphodiesterasen ist die maximale Gesamtkonzentration entsprechen niedriger anzusetzen.

Das Arzneimittel gemäß Ausführungsform (3) ist bevorzugt zur topischen, besonders bevorzugt zur transdermalen oder subcutanen Applikation geeignet.

Der Gehalt an Substanzen, die eine Steigerung des intrazellulären Spiegels von cGMP oder cGMP-Analoga bewirken, im Arzneimittel ist dabei vom Fachmann an die jeweilige Therapie und an den Patienten anzupassen. Ein Mittel gemäß Ausführungsform (4) kann zur Therapie von Pigmentstörungen oder Melanin-Stoffwechselstörungen verwendet werden. Dies geschieht durch Applikation einer effektiven Dosis des erfindungsgemäßen Mittels am tierischen oder menschlichen Patienten. Die Dosis sollte generell von 2,5 µg/cm² Haut/Tag bis 12,5 µg/cm² Haut/Tag betragen, bevorzugt von 7,5 µg/cm² Haut/Tag bis 10 µg/cm² Haut/Tag. Im Falle der spezifischen Inhibitoren von Phosphodiesterasen ist sie in der Regel deutlich niedriger anzusetzen, nämlich von 4 µg/cm² Haut/Tag bis 10 µg/cm² Haut/Tag, bevorzugt von 7 µg/cm² Haut/Tag bis 9 µg/cm² Haut/Tag.

Die Erfindung wird anhand der folgenden Beispiele illustriert, welche jedoch nicht limitierend für den Erfindungsgegenstand sein sollen.

### Ausführungsbeispiele

### Allgemein:

100 g einer Basiscreme wurde hergestellt aus 25,5 g weißer Vaseline, 7,5 g handelsüblichen mittelkettigen Triglyzeriden, 4,0 g Glycerinmonostearat und 6,0 g Cetylalkohol und einer Wasserphase aus 7,0 g Macrogol-1000 Glycerylmonostearat, 10,0 g Propylenglycol und 40,0 g Wasser.

Die in den Beispielen 1 bis 3 beschriebenen Cremes werden direkt auf die Haut aufgetragen.

Die Stammlösungen wurden wie folgt hergestellt:
100 mM YC-1 wurde in 50% DMSO verwendet.
100 mM 8-pCPT-cGMP wurde in 50% DMSO verwendet.
10 mM Trequinsin wurde in 100% DMSO verwendet.
Alle Verdünnungen wurden mit phosphate buffered saline (PBS), pH 7,4 durchgeführt.

### Beispiel 1:

Zu 100 g der Basiscreme wurden 22,5 ml einer 100 mM Stammlösung von 8-PCPT-cGMP (erhältlich von BIOLOG, Bremen, Deutschland; Endkonzentration 20 mM) bei Raumtemperatur (20 °C) unter gutem Umrühren beigemischt.

### Beispiel 2:

Zu 100 g der Basiscreme wurden 22,5 ml einer 10 mM Stammlösung von Trequinsin (Endkonzentration 2 mM) bei Raumtemperatur (20 °C) unter gutem Umrühren beigemischt.

### Beispiel 3:

Zu 100 g der Basiscreme wurden 22,5 ml einer 100 mM Stammlösung von 3-(5'-hydroxymethyl-2'-furyl)-1-benzylindazol (Endkonzentration 20 mM) bei Raumtemperatur (20 °C) unter gutem Umrühren beigemischt.

### Beispiel 4:

Humane epidermale Melanozyten (HEMn-MP, Cat. No. C-102-5C, Cascade Biologics, Mansfield, UK) wurden zuerst nach dem Protokoll des Herstellers in Medium 254 (Cat. No. M-254-500, Cascade Biologics, Mansfield, UK), welches PMA-freies HMGS-2 (Cat. No. S-016-5, Cascade Biologics, Mansfield, UK) enthielt, im Brutschrank (37°C, 5% CO₂, 95% Luftfeuchtigkeit) so lange expandiert, bis die benötigte Zellzahl für sechs T-75 Kulturflaschen (ca. 3x10⁶ Zellen per 75 cm²) erreicht war.

Zwei Tage vor dem Versuch zur Stimulation der Melanogenese wurde das komplette Medium durch MCDB 153 Medium (M7403, Sigma, Taufkirchen, Deutschland), welches 2% foetales Kälberserum und 30 µg/ml "bovine pituitary extract" enthielt, ersetzt. In drei der Kulturflaschen wurden zum Zeitpunkt 0 jeweils 150 µl 10 mM 8-PCPT-cGMP (cGMP-Analogon), gelöst in 20% Dimethylsulfoxid/PBS, zugegeben. Zu drei Kontrollkulturflaschen wurde nur das Lösungsmittel zugesetzt. Diese Prozedur wurde jeden Tag in einem Zeitraum von 4 Tagen wiederholt, d.h. das alte Medium wurde jeden Tag entsprechend durch neues MCDB Medium mit 8-PCPT-cGMP oder Lösungsmittel ersetzt. Die Zellen wurden nach einer 4-tägigen Inkubation mit jeweils 0,5 ml Trypsin/EDTA-Lösung (0,02%/0,005%) abgelöst. Die Reaktion wurde mit 0,5 ml 100% foetales Kälberserum gestoppt und die Zellen in 5 ml PBS aufgenommen. Die Zellsuspensionen wurden für 3 min bei 316 x g zentrifugiert. Die Zellzahl wurde mittels Coulter Counter (Coulter Electronics GmbH, Krefeld, Deutschland) ermittelt.

Die Bestimmung des Gesamt-Melaningehaltes wurde analog der Methode von Lee et al. (T.H. Lee et al., 1972, 91, 1180-1188) durchgeführt. Die Überstände der Zentrifugation wurden dazu verworfen und die Zellpellets, die jeweils ca. 3x10⁶ Zellen enthielten, wurden in jeweils 500 µl 2 N NaOH aufgenommen und über Nacht bei
- 80°C inkubiert. Danach wurden diese resuspendierten Homogenate für 2 h bei 90 °C inkubiert. Anschließend wurden die Proben für 15 min bei 17860 x g zentrifugiert. Die Überstände wurden in einem Spektrophotometer bei 400 nm gemessen. Der Melaningehalt wurde aus einer Standardkurve (250, 100, 175, 125, 100, 50, 15, 10, 5, 0 µg/ml Melanin), die aus einer Standardlösung (1 mg/ml synthetisches Melanin (Sigma, M8631, Taufkirchen, Deutschland)) erstellt wurde, ermittelt und in µg pro 10⁶ Zellen angegeben.

Ergebnis: der Melaningehalt der Zellen, die mit 8-PCPT-cGMP als cGMPanaloge Verbindung behandelt wurden, war im Vergleich zu den Kontrollen ohne 8-PCPT-cGMP um ca. 25% angestiegen. Dies zeigt, dass die Erhöhung von cGMP direkt die Melaninbildung stimuliert.

## Patentansprüche

1. Verfahren zum Bräunen der menschlichen oder tierischen Haut umfassend das Applizieren eines Mittels auf oder in die Haut , welches eine oder mehrere Substanzen enthält, die eine Steigerung des intrazellulären Spiegels von cGMP oder cGMP-Analoga bewirken.

2. Verfahren nach Anspruch 1, wobei die Substanzen ausgewählt sind aus der Gruppe umfassend cGMP, cGMP-Analoga, Inhibitoren von Phosphodiesterasen, Inhibitoren von Multidrug Resistance Proteins (MRPs), NO-unabhängigen Aktivatoren von Guanylatzyklase und die pharmazeutisch akzeptablen Salze derselben.

3. Verfahren nach Anspruch 1 oder 2, wobei das Mittel ein oder mehrere cGMP-Analoga enthält, die insbesondere ausgewählt sind aus 8-PCPT-cGMP (8-(4-chlorophenylthio)guanosine 3',5'-cyclic monophosphate), 8-Bromo-cGMP und Monobutyryl-3,5-cGMP.

4. Verfahren nach Anspruch 3, wobei das Mittel 8-PCPT-cGMP enthält.

5. Verfahren nach Anspruch 2, wobei das Mittel einen oder mehrere Inhibitoren von Phosphodiesterasen enthält, die insbesondere ausgewählt sind aus Koffein, 3-Isobutyl-1-Methylxanthin, Theophyllin, Zaprinast, Trequinsin, Sildenafil, Tadalafil und Vardenafil.

6. Verfahren nach Anspruch 5, wobei das Mittel Trequinsin enthält.

7. Verfahren nach Anspruch 2, wobei das Mittel einen oder mehrere Aktivatoren von Guanylatzyklase enthält, die insbesondere ausgewählt sind aus 3-(5'-hydroxymethyl-2'-furyl)-1-benzylindazol (YC-1), natriuretische Peptide wie h-BNP 32 (brain natriuretic peptide), h-CNP 22 (C-type natriuretic peptide), α-ANP (atrial natriuretic peptide), Guanylin und Uroguanylin.

8. Verfahren nach Anspruch 7, wobei das Mittel 3-(5'-hydroxymethyl-2'-furyl)-1-benzylindazol (YC-1) enthält.

9. Verfahren nach einem oder mehreren der Ansprüche 1 bis 8, wobei das Mittel auf die Haut, insbesondere als Creme, Salbe, Gel, Paste, Lotion oder Lösung, unverdünnt und/oder als Teil eines Pflasters appliziert wird.

10. Verwendung eines Mittels wie in einem oder mehreren der Ansprüche 1 bis 8 definiert zur Bräunung der menschlichen oder tierischen Haut.

11. Verwendung des Mittels wie in einem oder mehreren der Ansprüche 1 bis 8 definiert zur Herstellung eines Arzneimittels zur Therapie von Melanin-Stoffwechselstörungen oder Pigmentstörungen.

12. Verwendung nach Anspruch 11, wobei das Arzneimittel zur topischen, insbesondere zur transdermalen oder subcutanen Applikation geeignet ist.

13. Mittel zur Bräunung der Haut wie in einem oder mehreren der Ansprüche 1 bis 8 definiert.
